(19) Europäisches Patentamt
European Patent Office
Office européen des brevets

(11) Veröffentlichungsnummer: **0 121 764**
**B1**

(12) **EUROPÄISCHE PATENTSCHRIFT**

(45) Veröffentlichungstag der Patentschrift: **30.05.90**

(51) Int. Cl.[5]: **C 07 K 7/10,** A 61 K 37/02

(21) Anmeldenummer: **84102383.1**

(22) Anmeldetag: **05.03.84**

(54) **Polypeptide.**

(30) Priorität: **07.03.83 US 472700**

(43) Veröffentlichungstag der Anmeldung:
**17.10.84 Patentblatt 84/42**

(45) Bekanntmachung des Hinweises auf die Patenterteilung:
**30.05.90 Patentblatt 90/22**

(84) Benannte Vertragsstaaten:
**AT BE CH DE FR GB IT LI LU NL SE**

(56) Entgegenhaltungen:
**EP-A-0 117 034**
**EP-A-0 138 416**

**UNLISTED DRUGS, Band 35, Nr. 3, März 1983, page 41; "hpGRF"**
**Endocrin Soc., 65th Annual Meeting, Program and Abstract Book, p. 154 (1983)**

(73) Patentinhaber: **F. HOFFMANN-LA ROCHE AG**
**Postfach 3255**
**CH-4002 Basel (CH)**

(72) Erfinder: **Felix, Arthur M.**
**257 Central Avenue**
**West Caldwell N.J. 07006 (US)**
Erfinder: **Heimer, Edgar P.**
**87 Edison Terrace**
**Sparta N.J. 07871 (US)**

(74) Vertreter: **Lederer, Franz, Dr. et al**
**Van der Werth, Lederer & Riederer**
**Patentanwälte Lucile-Grahn-Strasse 22**
**D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**EP 0 121 764 B1**

**Beschreibung**

Kürzlich haben Dr. Guillemin und Mitarbeiter vom Salk Institut über die Isolierung, Synthese und biologische Aktivität einer Gruppe verwandter Substanzen, die sie Wachstumshormon-Releasing-Faktor (GRF: Science *218*, 585 [Nov. 5, 1982]) nannten, berichtet. Nach diesem Faktor wurde von den Wissenschaftlern seit vielen Jahren vergeblich gesucht. Die Suche war wegen der sehr geringen Mengen, in denen dieser Faktor in der Natur vorkommt, äusserst mühsam.

Die erfolgreiche Isolierung von GRF ist weitgehend eine Folge der Entdeckung, dass GRF in grossen Mengen von Pankreas-Tumoren, die bei Akromegalie vorkommen, produziert wird. Drei verschiedene Formen von GRF wurden in Pankreas-Tumoren beobachtet, bei denen es sich um homologe Peptide mit 44, 40 und 37 Aminosäuren handelt und die sich lediglich im Carboxylende voneinander unterscheiden. Das 44-Aminosäure-GRF besitzt eine amidierte Carboxylgruppe, während die anderen beiden Formen eine freie endständige Carboxylgruppe enthalten. Es zeigte sich, dass die Entfernung der Amidgruppe des 44-Aminosäure-GRF eine erhebliche Verringerung der biologischen Aktivität bewirkt.

Die amidierte Form von GRF(1—44) ist offenbar das Grundmolekül und soll die höchste biologische Aktivität in vitro besitzen. Alle drie Peptide sind jedoch in vivo von etwa gleicher Aktivität. Es konnte ferner gezeigt werden, dass bei Entfernung der aminoendständigen Aminosäure Tyrosin GRF seine Bioaktivität vollständig verliert.

Kürzlich haben Rivier et al. in Nature *300*, 276—278 (18 November, 1982) berichtet, dass synthetisch hergestelltes GRF(1—29)-$NH_2$, GRF(1—32)-$NH_2$, GRF(1—39)-$NH_2$, GRF(1—40)-$NH_2$, GRF(1—40)-Phe-$NH_2$, GRF(1—40)-Phe-OH und GRF(1—40)-Phe-Gln-$NH_2$ ähnliche Aktivitäten wie GRF(1—40)-OH besitzen (innerhalb eines Faktors von 2).

Man glaubt, dass das Wachstum bei Tieren durch eine Anzahl von nacheinander geschalteten Regulatormolekülen reguliert wird. Im Hypothalamus wird GRF erzeugt, das auf die Hypophyse wirkt, wo dann das Wachstumshormon ausgeschüttet wird. Die Hypophyse steht unter einer negativen Rückkopplungskontrolle durch Somatostatin und Insulin-Wachstumsfaktor (IGF). Es stellte sich heraus, dass GRF ausserordentlich aktiv ist, mit einer $ED_{50}$ von etwa 50 fMol/ml oder 75 pg/ml, und die Ausschüttung von Microgramm/ml-Mengen von Wachstumshormon in das Blut auslöst. GRF kann daher therapeutisch fast überall dort angewandt werden, wo bisher eine Behandlung mit Wachstumshormon stattfand. Beispiele solcher therapeutischer Behandlungen sind die Behandlung von hypophysärem Zwergwuchs, durch Unregelmässigkeiten der Wachstumshormon-Produktion bedingter Diabetes, Beschleunigung der Wundheilung, Behandlung von Verbrennungen und die Verzögerung des Alterungsprozesses. Im Hinblick auf seine vorteilhafte Aktivität verglichen mit dem Wachstumshormon selbst, wird GRF auch vorteilhaft in der Landwirtschaft eingesetzt werden können. Anwendungen in der Landwirtschaft sind beispielsweise die Stimulierung der Entwicklung von Geflügel oder Tieren, die der Fleischproduktion dienen und die entweder früher geschlachtet bzw. mit einer geringeren Futtermenge aufgezogen werden können.

Ausserdem dürfte GRF auch eine Stimulierung der Milchproduktion bei Milchkühen und erhöhte Eierproduktion bei Hennen bewirken.

GRF in seinen verschiedenen Formen hat eine Molekülgrösse, die die Herstellung sowohl durch Festphasen- wie durch Flüssigphasen-Peptidsynthese ermöglicht. Andererseits ist davon auszugehen, dass eine ökonomische Grossproduktion dieser therapeutisch wertvollen Substanzen zweckmässigerweise durch rekombinante DNA-Technologie erfolgt. Unter Anwendung bekannter rekombinanter DNA-Techniken kann eine DNA-Sequenz, die das Strukturgen für GRF enthält, in einen replikablen Expressionsvektor eingebaut werden, so dass sie sich unter der Kontrolle geeigneter Elemente wie Promotor-Operator-Sequenzen und Ribosomenbindungssequenzen befindet. Mit den Expressionsvektoren können dann Mikroorganismen, beispielsweise Bakterien, oder Säugetierzellen transformiert und so gezüchtet werden, dass sie GRF exprimieren.

Andererseits gibt es möglicherweise Probleme bei der Herstellung von GRF mittels rekombinanter DNA-Technologie. So ist z.B. festgestellt worden, dass Polypeptide von ähnlicher Molekülgrösse wie GRF eher von Proteasen, die in Bakterien wie E. coli vorhanden sind, abgebaut werden als grössere Proteinmoleküle. Darüberhinaus arbeitet der zelluläre Apparat, der die Transkription und Translation reguliert, aus Gründen, die noch nicht völlig verstanden werden, offensichtlich wirkungsvoller bei längeren DNA-Sequenzen, wodurch es schwieriger ist akzeptable Expressionsniveaus bei kleineren Polypeptiden, wie z.B. GRF, zu erreichen. Ein weiteres noch zu lösendes Problem der Herstellung von rekombinantem GRF ist das seiner Reinigung und Abtrennung von anderen, im Wirtsbakterium synthetisierten Proteinen und Endotoxinen.

Es ist vorgeschlagen worden, die GRF kodierende DNA-Sequenz an eine DNA-Sequenz, die ein längeres Proteinmolekül kodiert über Phosphodiesterbindungen zu koppeln und die so erhaltene DNA-Sequenz in einen Expressionsvektor einzubauen, so dass ein Fusionsprotein exprimiert wird. Das Fusionsprotein wäre dem Abbau durch bakterielle Proteasen weniger zugänglich als GRF. Man könnte als Fusionspartner ein Protein auswählen, das in bestimmten Expressionsvektoren sehr gut exprimiert werden kann, beispielsweise ein Interferon, und dadurch eine hohe Expression des Fusionsproteins erreichen. Darüberhinaus gibt es monoklonale Antikörper, die selektiv Interferon erkennen und binden und man könnte das GRF-Interferonfusionsprotein an einer solchen monoklonalen Antikörpersäule mit einem

geeigneten Lösungsmittel reinigen. Nach der Reinigung muss das GRF aus dem Fusionsprotein abgespalten und isoliert werden. Die einfachste Methode ist die Spaltung mit CNBr. Da CNBr selektiv am Carboxylende eines Methoninrests spaltet ist es notwendig, eine DNA-Sequenz zu konstruieren, die zwischen der letzten, Carboxyl-endständigen Aminosäure des Fusionspartners, beispielsweise des Interferons, und der ersten Amino-endständigen Aminosäure des GRF ein Methonin enthält.

Die vorstehend beschriebene Strategie kann jedoch für die Herstellung von GRF zunächst nicht angewendet werden, da GRF, wie von Guillemin gefunden, in Position 27 einen Methoninrest enthält. Bei der CNBr-Spaltung des Fusionsproteins würde daher das Molekül auch an dieser Stelle gespalten werden. Die vorliegende Erfindung basiert nun auf der Entdeckung, dass der Methoninrest in Position 27 des GRF-Moleküls durch andere Aminosäuren ersetzt werden kann, ohne dass das Molekül dadurch seine spezifische Aktivität (Freisetzung von Wachstumshormon) verliert. Es wurde insbesondere gefunden, dass ein Polypeptid mit der Aminosäuresequenz des GRF, in dem der Methoninrest in Position 27 durch Leucin ersetzt worden ist, volle biologische Aktivität besitzt. Ferner wurde überraschenderweise gefunden, dass das am Carboxylende freie, d.h. nicht amidierte GRF mit 44-Aminosäuren und Leucin in Position 27 eine mindestens ebenso grosse biologische Aktivität aufweist wie das natürliche, am Carboxylende amidierte GRF(1—44).

Gemäss vorliegender Erfindung können also Polypeptide mit GRF-Aktivität mittels der vorstehend beschriebenen rekombinanten DNA-Technologie hergestellt werden, ohne dass sie durch CNBr gespalten werden und ohne dass sie in einer letzten Stufe amidiert werden müssen um die volle biologische Aktivität zu erhalten.

Die erfindungsgemässen Leucin- analogen des natürlichen GRF besitzen gegenüber natürlichem GRF wahrscheinlich den Vorteil erhöhter Stabilität, da Leucin nicht wie Methionin zum Sulfoxid oxidiert wird.

Der in der vorliegenden Beschreibung und den Ansprüchen verwendete Ausdruck "GRF" bezieht sich auf den menschlichen Wachstumshormon-Releasing-Faktor, der ein Polypeptid mit der folgenden Aminosäuresequenz ist (Science *218*, 585, November 5, 1982)

```
1           5                  10                 15
Tyr-Ala-Asp-Ala-Ile-Phe-Thr-Asn-Ser-Tyr-Arg-Lys-Val-Leu-Gly-

                20                 25                 30
Gln-Leu-Ser-Ala-Arg-Lys-Leu-Leu-Gln-Asp-Ile-Met-Ser-Arg-Gln-

                35                 40
Gln-Gly-Glu-Ser-Asn-Gln-Glu-Arg-Gly-Ala-Arg-Ala-Arg-Leu
```

sowie auf Fragment hiervon, die mindestens die ersten 28 Aminosäuren des Polypeptides enthalten und Wachstumshormon-Releasing-Aktivität aufweisen. Werden im Zusammenhang mit GRF die Suffixe "—OH" und "—$NH_2$" verwendet so bedeuten sie die freie bzw. amidierte Form. Wird kein Suffix verwendet, so umfasst der Ausdruck GRF beide Formen, d.h. die freie Säure und die amidierte Form. GRF-Analoge werden durch Angabe derjenigen Aminosäure, die an einer bestimmten Stelle der Sequenz die dort natürlicherweise vorkommende Aminosäure ersetzt, vor der Bezeichnung "GRF", gekennzeichnet. So bezeichnet z.B. der Ausdruck "($Leu^{27}$)-GRF" ein Polypeptid mit der Aminosäuresequenz vo GRF (mit freiem oder amidiertem Carboxylende), in dem in Position 27 der Methioninrest durch Leucin ersetzt wurde. Zahlen, die der Bezeichnung "GRF" in runden Klammern folgen, bezeichnen die Länge der Aminosäuresequenz des Moleküls; so bedeutet beispielsweise GRF(1—40) ein GRF-Molekül, das aus den ersten 40 Aminosäuren der vollen Sequenz des natürlichen GRF besteht.

Die vorliegende Erfindung betrifft Polypeptide mit der Aminosäuresequenz von mindestens den ersten 28 Aminosäuren von GRF, wobei der Methioninrest in Position 27 durch eine andere Aminosäure substituiert wurde. Diese Aminosäure ist so gewählt, dass das Polypeptid nicht durch Cyanbromid gespalten wird, andererseits die volle Wachstumshormon-Releasing-Aktivität behält. Geeignete Aminosäuren, die für die Substitution in Frage kommen, sind solche, die dem Methionin strukturell ähnlich sind aber keine Spaltstellen für Cyanbromid darstellen, d.h. Leucin, Isoleucin und Valin. Im Zusammensetzung mit der vorliegenden Erfindung ist Leucin bevorzugt, weil es zu GRF-Analogen führt, die bei freier endständiger Carboxylgruppe volle Wachstumshormon-Releasing-Aktivität besitzen. Von besonderem Interesse sind $Leu^{27}$-Analoge des GRF mit einer Sequenz von 41—44 Aminosäuren.

Die erfindungsgemässen Polypeptide können durch rekombinante DNA-Technologie (wie bereits oben ausgeführt) oder durch konventionelle Synthese in fester oder flüssiger Phase hergestellt werden. ($Leu^{27}$)-GRF(1—44)-OH, die interessanteste Verbindung, wurde durch Festphasensynthese unter Verwendung von 4-(Hydroxymethyl)-phenylacetamidomethyl(=PAM)-poly(styrol-co-divinylbenzol)-Harz als Träger hergestellt. Das Polypeptid wurde durch präparative HPLC gereinigt. Dass es sich um homogenes Material handelte, konnte in zwei analytischen HPLC-Systemen, durch isoelektrische Fokussierung und Hochspannungsdünnschichtelektrophorese gezeigt werden. Ferner zeigte die Analyse die erwartete Aminosäurezusammensetzung. Das entsprechende Amid, ($Leu^{27}$)-GRF(1—44)-$NH_2$ wurde in analoger Weise unter Verwendung des Benzhydrylaminharzes als Träger durch Festphasensynthese hergestellt. Der

Fachmann weiss, dass bei Verwendung von PAM-Harz als Träger die Behandlung mit HF als letzte Stufe ein Polypeptid mit freier Carboxylgruppe liefert, während die gleiche Behandlung bei Verwendung eines Benzhydrylaminharzes ein Polypeptid mit carboxylendständiger Amidgruppe liefert.

Die Reinigung der erfindungsgemässen Polypeptide kann nach in der Peptid-Chemie bekannten Methoden erfolgen. Wie bereits erwähnt, können die nach der Festphasensynthese erhaltenen Peptide durch präparative HPLC gereinigt werden. Andere chromatographische Verfahren wie Gelpermeation, Ionenaustausch und Verteilungschromatographie können jedoch ebenfalls verwendet werden.

Die erfindungsgemässen Polypeptide besitzen Wachstumshormon-Releasing-Aktivität. Dementsprechend können sie zur Behandlung von wachstumsbedingten Umregelmässigkeiten wie hypophysärem Zwergwuchs oder Diabetes, der durch Störungen in der Wachstumshormon-Produktion bedingt ist, verwendet werden. Sie können ebenfalls eingesetzt werden zur Erhöhung des Wachstums bei Tieren für die Fleischproduktion.

Geeignete Dosierungsmengen der erfindungsgemässen Polypeptide hängen von den Umständen und dem zu behandelnden Individuum ab. Der Fachmann wird in der Lage sein, die geeignete Menge selbst zu bestimmen aufgrund der bei normalem Wachstum vorhandenen Konzentrationen an Wachstumshormon und der Wachstumshormon-Releasing-Aktivität des Polypeptids. Da bei Verabreichung von $(Leu^{27})$-GRF-OH in vitro eine um den Faktor von mindestens 10 grössere Menge an Wachstumshormon bezogen auf $(Leu^{27})$-GRF-OH ausgeschüttet wird, ist es klar, dass wesentlich geringere Mengen an erfindungsgemässen Releasing-Faktoren gegenüber dem erforderlichen Wachstumshormon eingesetzt werden können. Die im Zusammenhang mit der Behandlung von Wachstumsstörungen notwendigen Dosen variieren ebenfalls von Individuum zu Individuum in Abhängigkeit von der Schwere des Mangels an Wachstumshormon. Im allgemeinen wird eine Menge von etwa 0,5 µg/kg Körpergewicht ausreichen, um die gewünschte Ausschüttung an Wachstumshormon zu bewirken. Zur Anregung eines überdurchschnittlichen Wachstums beim Vieh müssen dagegen erheblich höhere Mengen (pro kg Körpergewicht) aufgewendet werden als bei der Behandlung von hormonalen Wachstumsstörungen wie dem hypophysären Zwergwuchs beim Menschen.

Der normale Wachstumshormon-Spiegel variiert nicht nur beträchtlich von Individuum zu Individuum, sondern bei einem bestimmten Individuum auch beträchtlich innerhalb des Tagesverlaufs. Der normale Serumspiegel des Wachstumshormons liegt bei Erwachsenen bei 0—10 ng/ml, bei Kindern bei 0—20 ng/ml.

Die Behandlung von hypophysärem Zwergwuchs mit $(Leu^{27})$-GRF erfolgt zweckmässigerweise während der normalen Wachstumsperiode. Während sie bei Frauen nicht wesentlich über den Zeitpunkt des Beginns der Menses, also etwa das zwölfte bis sechzehnte Lebensjahr, hinaus erfolgen sollte, kann sie bei Männern normalerweise bis zum achtzehnten oder neunzehnten, in Ausnahmefällen bis zum fünfundzwanzigsten Lebensjahr erfolgen.

Die vorliegende Erfindung betrifft ferner eine Methode zur Erhöhung des Wachstums von Tieren durch Verabreichung von $(Leu^{27})$-GRF in Mengen, die die Produktion von Wachstumshormon auf ein Niveau heben, das über demjenigen liegt, das für normales Wachstum notwendig ist.

Die erfindungsgemässen Polypeptide können in Form von pharmazeutischen Präparaten, wie sie in der Human- oder Veterinär-Medizin üblich sind, verabreicht werden. Die Präparate können in an sich bekannter Weise hergestellt werden. Die Verbindungen eignen sich zur intravenösen, subkutanen, intramuskulären oder intraperitonealen Verabreichung. Eine geeignete Dosierungsform für pharmazeutische Zwecke liegt bei etwa 0,01 bis 0,5 mg $(Leu^{27})$-GRF-OH, beispielsweise in lyophilisierter Form, geeignet zur Rekonstitution mit sterilem Wasser oder Kochsalzlösung. Das Präparat sollte aus Gründen der Stabilität des $(Leu^{27})$-GRF-OH bei einem pH unterhalb von 6,0 gehalten werden. In den Präparaten kann Serumalbumin derjenigen Spezies, die behandelt werden soll (z.B. humanes Serumalbumin für Menschen, Rinder-Serumalbumin bei Kühen, usw.), anwesend sein, gegebenenfalls in Gegenwart anderer bekannter pharmazeutischer Adjuvantien.

Die folgenden Beispiele illustrieren die Erfindung ohne sie zu beschränken. Sofern nicht anders angegeben, handelt es sich bei der Angabe von Prozenten oder Teilen stets um GEwichtseinheiten. Alle Temperaturen sind in Grad Celsius angegeben. Es wurden optisch aktive geschützte Aminosäuren mit L-Konfiguration verwendet. Die geschützten Aminosäuren wurden dünnschichtchromatographisch auf Kieselgel mit dem System Chlor-TDM geprüft. Es wurden die folgenden Abkürzungen für die Schutzgruppen verwendet:

BOC=t-Butyloxycarbonyl
Z=Benzyloxycarbonyl
2ClZ=2-Chlorbenzyloxycarbonyl
Bzl=Benzyl
2,6-$Cl_2$-Bzl=2,6-Dichlorbenzyl
Tos=p-toluolsulfonyl

Beispiel

Herstellung von $(Leu^{27})$-GRF(1—44)-OH

$(Leu^{27})$-GRF(1—44)-OH wurde durch sukzessive Kopplung der einzelnen Aminosäuren unter Verwendung eines im Handel erhältlichen Automaten für Festphasen-Peptidsynthese hergestellt. Es

wurden $N^{\alpha}$-Boc-Aminosäuren verwendet. Trifunktionelle Aminosäuren waren folgendermassen geschützt: $N^{\alpha}$-Boc-Lys(2ClZ), $N^{\alpha}$-Boc-Asp(OBzl), $N^{\alpha}$-Boc-Glu(OBzl), $N^{\alpha}$-Boc-Ser(Bzl), $N^{\alpha}$-Boc-Thr(Bzl), $N^{\alpha}$-Boc-Tyr(2,6-$Cl_2$-Bzl).

Boc-Leu-4-(Oxymethyl)-phenylacetamidomethyl-Harz wurde durch Kopplung von Boc-Leu-4-(Oxymethyl)-phenylessigsäure an Aminomethyl-polystyrol-co-divinylbenzol-Harz (10 g, 0,714 mMol/g), wie in J. Org. Chem. *43*, 2845—2852 (1978) beschrieben, hergestellt. Der Substitutionsgrad des Harzes betrug 0,135 mMol/g Polystyrol-co-divinylbenzol. Das Boc-Leu-Harz wurde dann in das Reaktionsgefäss eines Syntheseautomaten gebracht, wo die weiteren Aminosäuren sukzessive angehängt wurden unter jeweiliger Abspaltung der aminoterminalen Schutzgruppe. In jeder Reaktionsstufe uwrde mit einem vierfachen Überschuss an Dicyclohexylcarbodiimid (DCC) und der entsprechenden Boc-Aminosäuren gearbeitet, wobei die Abspaltung der Boc-Gruppe durch Behandlung mit 50%iger Trifluoressigsäure (TFA) in Methylenchlorid bewerkstelligt wurde.

Ausgehend von 10 g Boc-Leu-4-(Oxymethyl)-phenylacetamidomethyl-Harz wurde in jedem Kopplungszyklus die folgenden Massnahmen durchgeführt:

(1) Behandlung mit 50%iger TFA in Methylenchlorid während einer Minute;
(2) Behandlung mit frischer 50%iger TFA in Methylenchlorid während 20 Minuten;
(3) WAschen mit Methylenchlorid (viermal je 1 Minute);
(4) Waschen mit 8% Diisopropylethylamin (DIEA) in Methylenchlorid während 2 Minuten;
(5) WAschen mit Methylenchlorid während 1 Minute;
(6) Wiederholung der Massnahmen 4 und 5;
(7) Waschen mit 2-Propanol (zweimal je 1 Minute);
(8) Waschen mit Methylenchlorid (sechsmal je 1 Minute);
(9) Umsetzung mit 4 Aquivalenten Boc-Aminosäure in Methylenchlorid (50 ml/g) während 5 Minuten, anschliessend mit 4 Äquivalenten DCC während 20 Minuten;
(10) Behandlung mit 1% DIEA in Methylenchlorid während 10 Minuten;
(11) WAschen mit Methylenchlorid während 2 Minuten;
(12) Wiederholung der Massnahmen 10 und 11;
(13) Waschen mit Methylenchlorid (sechsmal je 2 Minuten).

In folgenden Fällen wurde vom vorstehenden Protokoll abgewichen:

Boc-Gln-OH wurde als symmetrisches Anhydrid (6 Äquivalente in Dimethylformamid (DMF)) gekoppelt mit anschliessendem schnellen Waschen und Neutralisation nach Entfernung der Boc-Gruppe, um die Bildung von Pyrrolidoncarbonsäure (Pca) möglichst gering zu halten. Der ungeschützte Gln-Rest wurde dann mit dem symmetrischen Anhydrid der nächsten Aminosäure in DMF gekoppelt, ebenfalls um die Bildung von Pca auf ein Minimum zu reduzieren. Boc-Asn-OH (6 Äquivalente in DMF) wurde während 1 Stunde nach der DCC-1-Hydroxybenzotriazol-Methode (Chem. Ber. *103*, 788—798 (1979) und Int. J. Peptide Protein Res. *7*, 495—501 (1975)) zwecks Reduktion von Nitril-Bildung gekoppelt. Nach der Kopplung von Ser(Bzl) in Position 28 wurde 1 g des Peptid-Harzes (schätzungsweise etwa 100 µMol) für die Synthese von ($Leu^{27}$)-GRF-OH verwendet, während der Rest zur Herstellung anderer GRF-Analoga in denen die Position 27 durch andere Aminosäuren eingenommen wird, verwendet wurde.

Die Effizienz der Kopplung wurde nach jedem Zyklus mit der Ninhydrin-Methode (Analytical Biochem. *34*, 595—598 (1970) überprüft und im allgemeinen als 100%ig nach jeweils 2 Kopplungen befunden. Ausnahmen, die mehrmalige Kopplung erforderlich machten, waren die folgenden: (1) Boc-Arg(Tos) in 10% DMF-$CH_2Cl_2$ an $Ala^{42}$, $Gly^{39}$ und $Lys^{12}$; (2) Boc-Lys(2-ClZ) an $Val^{13}$; (3) Boc-Asp(OBzl) an $Ile^{26}$; (4) Boc-Leu an $Leu^{23}$ und (5) Boc-Ser(Bzl) an $Arg^{29}$. Die Boc-Gruppe der letzten Aminosäure wurde nach der in J. Am. Chem. Soc. *98*, 2324—2328 (1976) beschriebenen Methode abgespalten und das Peptid-Harz wurde getrocknet. Das so erhaltene, Seitenketten-geschützte Peptid-harz (etwa 80 µMol) wurde mit wasserfreiem flüssigem HF behandelt, wodurch die Schutzgruppen der Seitenketten abgetrennt und das Peptid vom Harz abgespalten wurde. Es wurde mit einem Gemisch aus p-Cresol (10%), Dimethylsulfit (65%) und HF (25%) bei 0°C während einer Stunde und anschliessend mit p-Cresol (10%) und HF (90%) bei 0°C während 2 Stunden behandelt. Nach Aufarbeitung wurden 332 mg rohes ($Leu^{27}$)-GRF-OH erhalten. Ein Teil dieses Materials (50 mg) wurde durch semipräparative HPLC unter Verwendung einer Whatman Partisil® M-9 ODS-3-Säule (0,94×50 cm) gereinigt. Die Säule wurde mit 0,5% TFA/$H_2O$/$CH_3CN$ und einem linearen Gradienten von 30—45% $CH_3CN$ während 2 Stunden bei einer Durchflussmenge von 3 ml/Min. eluiert. Es wurden Fraktionen von jeweils 3 ml gesammelt und aliquote Teile davon mittels analytischer HPLC untersucht. Das gewünschte Produkt wurde in den Minuten 56 bis 58 eluiert, die Fraktionen wurden kombiniert, eingeengt und lyophilisiert. Der Rest des Rohmaterials wurde in der gleichen Weise gereinigt und lieferte 16,8 mg. Die seitlich angrenzenden Fraktionen wurden ebenfalls zusammengefasst, eingeengt, lyophilisert und rechromatogrpahiert, wobei insgesamt 18,5 mg reines Material (5%) erhalten wurde. Das gereinigte Materiael war homogen in 2 analytischen HPLC-Systemen, wanderte bei der isoelektrischen Fokussierung an die Kathode und war homogen in der Hochspannungsdünnschichtelektrophorese ($R_{Arg}$=0,18). Die Chromatographie des tryptischen Abbauproduktes einer Probe lieferte die erwarteten 6 grösseren Fragmente (1—11), (13—20), (22—29), (30—38), (39—41) und (42—43). Zusätzliche Fragmente können durch unvollständigen tryptischen Abbau entstehen.

$Leu^{27}$-Analoga von biologisch aktiven Fragmenten des GRF, z.B. ($Leu^{27}$)-GRF (1—40), ($Leu^{27}$)-GRF (1—39), ($Leu^{27}$)-GRF (1—32) oder ($Leu^{27}$)-GRF (1—29) können in analoger Weise dadurch hergestellt

werden, dass man die letzte, carboxylendständige Aminosäure der gewünschten Verbindung an das Harz koppelt und den Synthese-Automaten entsprechend programmiert. Zur Herstellung von beispielsweise ($Leu^{27}$)-GRF (1—32) wird Glycin an das Harz gekoppelt und die erste Aminosäure die der Syntheseautomat anhängt ist Glutamin.

Die biologische Aktivität von ($Leu^{27}$)-GRF(1—44)-OH wurde mit derjenigen von natürlichem GRF(1—44)-$NH_2$ verglichen, das aus einem Pankreastumor eines an Akromegalie leidenden Patienten isoliert wurde (Salk Institute Standard hp-GRF-$NH_2$(NL-A-10)). Der Test auf biologische Aktivität, bei dem die Fähigkeit gemessen wird, in Gewebekultur gezüchtete Zellen von Rattenhypophysen zur Wachstumshormonproduktion zu stimulieren, wurde in der folgenden Weise durchgeführt:

Die Hypophysen-Vorderlappen von 30—40 männlichen Sprague-Dawley-Ratten (175 g) wurden dreimal mit sterilem Hepes-Puffer (pH 7,5) gewaschen und bei 37°C in 20—30 ml Hepes-Puffer (0,025 m, pH 7,35), enthaltend Kollagenase (4 mg/ml) und Dispase (2 mg/ml), dispergiert. Nach 100—110 minütiger Behandlung mit einem Vortex-Mixer und Behandlung mit Pasteur-Pipette, wurden die dispergierten Zellen durch Zentrifugieren (4 Minuten, 150×g) abgetrennt und während 10 Minuten in Hepes-Puffer, enthaltend 8 µg/ml Neuraminidase und 200 µg/ml Ethylendiamintetraessigsäure-dinatriumsalz, pH 7,35, resuspendiert. Die Zellen wurden zweimal mit folgendem Medium gewaschen: F-12/DMEM/BGjb (6:3:1) (Gibco: 430—1700/430—1600/320—2591) mit 2 g/l Rinderserumalbumin (BSA), 2×38 g/l Hepes und 50 mg/l Gentamycin und auf Mikrotiterplatten aufgebracht ($1{,}5\times10^5$ Zellen/ml). Dem Medium in den einzelnen Löchern wurde entweder ($Leu^{27}$)-GRF(1—44)-OH oder natürliches GRF(1—44)-$NH_2$ in solchen Mengen zugesetzt, dass sich folgende Konzentrationen ergaben: 3.1, 6.3, 12.5, 25.0, 50.0, 100.0 und 200.0 fMol/ml. Die Kontrollen wurden nicht supplementiert. Das Aufbringen mit diesem Medium erfolgte unter Zusatz von 2% fötalem Kälberserum, um eine schnelle Fixierung der Zellen zu erreichen. Am vierten Tag wurden die Zellen zweimal mit dem oben definierten Medium (ohne Kälberserum) gewaschen. Schliesslich wurden jedem Loch 900 µl des Mediums sowie 100 µl Medium mit der Testsubstanz zugesetzt (jeweils dreifach). Nach dreistündiger Inkubation wurde der Überstand gesammelt und soweit verdünnt, dass Radioimmunoassays (RIAs) auf Ratten-Wachstumshormon durchgeführt werden konnten. Die RIAs wurden mit Sinhas anti-Maus-Wachstumshormon-Immunserum durchgeführt.

Die Resultate der Assays mit natürlichem GRF(1—44)-$NH_2$ und ($Leu^{27}$)-GRF(1—44)-OH sind in der folgenden Tabelle zusammengefasst:

GHR-Aktivität von GRF(1-44)-$NH_2$ (A) und ($Leu^{27}$)-GRF(1-44)-OH (B)

| Dosis [fmol/ml] | Wachstumshormon-Ausschüttung [ng/ml] A | B | Prozent der Kontrolle A | B |
|---|---|---|---|---|
| Kontrolle | 82 | 82 | - | - |
| 3.1 | 117 | 133 | 143 | 162 |
| 6.3 | 158 | 167 | 193 | 204 |
| 12.5 | 210 | 223 | 256 | 272 |
| 25.0 | 261 | 270 | 318 | 329 |
| 50.0 | 343 | 342 | 418 | 417 |
| 100.0 | 433 | 423 | 528 | 516 |
| 200.0 | 522 | 532 | 637 | 649 |

Aus den Werten der Tabelle geht hervor, dass die Aktivität von ($Leu^{27}$)-GRF(1—44)-OH gleich oder leicht höher ist als die von GRF(1—44)-$NH_2$. Die relative Aktivität von ($Leu^{27}$)-GRF(1—44)-OH gegenüber dem Standard des Salk Instituts wurde durch Computeranalyse obiger Daten festgestellt und beträgt 1,063.

**Patentansprüche für die Vertragsstaaten: BE CH DE FR GB IT LI LU NL SE**

1. Polypeptide der allgemeinen Formel

$$(Leu^{27})\text{-GRF}$$

worin GRF mindestens die ersten 28 Aminosäuren der vollen Sequenz des natürlichen menschlichen Wachstumshormon-Releasing-Faktors bedeutet und wobei das Carboxylende des Moleküls in freier Form oder als Amid vorliegt.

2. Ein Polypeptid gemäss Anspruch 1, wobei GRF die ersten 41 bis 44 Aminosäuren des natürlichen menschlichen Wachstumshormon-Releasing-Faktors umfasst.

3. Ein Polypeptid gemäss Anspruch 1 mit der Formel $(Leu^{27})$-GRF(1—44)-OH.

4. Ein Polypeptid gemäss Anspruch 1 mit der Formel $(Leu^{27})$-GRF(1—44)-$NH_2$.

5. Ein Polypeptid gemäss einem der Ansprüche 1—4 zur Anwendung als therapeutischer Wirkstoff.

6. Ein Polypeptid gemäss einem der Ansprüche 1—4 zur Anwendung als Wachstumsregulator beim Menschen.

7. Ein Polypeptid gemäss einem der Ansprüche 1—4 zur Anwendung als Wachstum-förderndes Mittel bei Tieren.

8. Verfahren zur Herstellung von Polypeptiden der allgemeinen Formel

$$(Leu^{27})\text{-GRF}$$

worin GRF mindestens die ersten 28 Aminosäuren der vollen Sequenz des natürlichen menschlichen Wachstumshormon-Releasing-Faktors bedeutet und wobei das Carboxylende des Moleküls in freier Form oder als Amid vorliegt, dadurch gekennzeichnet, dass man

(a) die Schutzgruppen eines geschützten Polypeptids entsprechender Aminosäuresequenz, das nach der Flüssigphasenmethode synthetisiert wurde, in an sich bekannter Weise abspaltet oder

(b) ein an ein Trägerharz gebundenes geschütztes Polypeptid entsprechender Aminosäuresequenz, das nach der Festphasenmethode synthetisiert wurde, mit wasserfreiem flüssigem HF behandelt.

9. Pharmazeutische Präparate zur parenteralen Applikation, gekennzeichnet durch einen therapeutisch wirksamen Gehalt an einem Polypeptid gemäss einem der Ansprüche 1—4 und ein inertes Trägermaterial.

10. Präparate gemäss Anspruch 9, dadurch gekennzeichnet, dass das Trägermaterial Human-Serumalbumin ist.

**Patentansprüche für den Vertragsstaat: AT**

1. Verfahren zur Herstellung von Polypeptiden der allgemeinen Formel

$$(Leu^{27})\text{-GRF}$$

worin GRF mindestens die ersten 28 Aminosäuren der vollen Sequenz des natürlichen menschlichen Wachstumshormon-Releasing-Faktors bedeutet und wobei das Carboxylende des Moleküls in freier Form oder als Amid vorliegt, dadurch gekennzeichnet, dass man

(a) die Schutzgruppen eines geschützten Polypeptids entsprechender Aminosäuresequenz, das nach der Flüssigphasenmethode synthetisiert wurde, in an sich bekannter Weise abspaltet oder

(b) ein an ein Trägerharz gebundenes geschütztes Polypeptid entsprechender Aminosäuresequenz, das nach der Festphasenmethode synthetisiert wurde, mit wasserfreiem flüssigem HF behandelt.

2. Ein Verfahren gemäss Anspruch 1 zur Herstellung von GRF das die ersten 41 bis 44 Aminosäuren des natürlichen menschlichen Wachstumshormon-Releasing-Faktors umfasst.

3. Ein Verfahren gemäss Anspruch 1 zur Herstellung von $(Leu^{27})$-GRF(1—44)-OH.

4. Ein Verfahren gemäss Anspruch 1 zur Herstellung von $(Leu^{27})$-GRF(1—44)-$NH_2$.

5. Pharmazeutische Präparate zur parenteralen Applikation, gekennzeichnet durch einen therapeutisch wirksamen Gehalt an einem Polypeptid, das nach einem der Verfahren der Ansprüche 1—4 hergestellt worden ist und ein inertes Trägermaterial.

6. Präparate gemäss Anspruch 5, dadurch gekennzeichnet, dass das Trägermaterial Human-Serumalbumin ist.

7. Verwendung eines Polypeptids, das nach einem der Verfahren der Ansprüche 1—4 hergestellt worden ist, zur Anwendung als Wachstums-förderndes Mittel bei Tieren.

**Revendications pour les Etats contractants: BE CH DE FR GB IT LI LU NL SE**

1. Polypeptides de formule générale

$$(Leu^{27})\text{-GRF}$$

dans laquelle GRF représente au moins les 28 premiers amino-acides de la séquence complète du facteur déclenchant la sécrétion de l'hormone de croissance humaine naturelle et dans laquelle l'extrémité carboxy de la molécule est sous forme libre ou sous forme d'amide.

2. Polypeptide selon la revendication 1, dans lequel GRF comprend les 41 à 44 premiers aminoacides du facteur déclenchant la sécrétion de l'homme de croissance humaine naturelle.

3. Polypeptide selon la revendication 1, de formule (Leu$^{27}$)-GRF(1—44)-OH.

4. Polypeptide selon la revendication 1 de formule (Leu$^{27}$)-GRF(1—44)-$NH_2$.

5. Polypeptide selon l'une des revendications 1—4 pour l'application en tant que substance active thérapeutique.

6. Polypeptide selon l'une des revendications 1—4 pour l'application chez l'homme en tant que régulateur de croissance.

7. Polypeptide selon l'une des revendications 1—4 pour l'application en tant que moyen accélérateur de croissance chez les animaux.

8. Procédé de préparation de polypeptides de formule générale

(Leu$^{27}$)-GRF

dans laquelle GRF représente au moins les 28 premiers amino-acides de la séquence complète du facteur déclenchant la sécrétion de l'homme de croissance humaine naturelle et dans laquelle l'extrémité carboxy de la molécule est sous forme libre ou sous forme d'amide, caractérisé en ce qu'

a) on élimine de la manière connue en soi les groupes protecteurs d'un polypeptide protégé de séquence correspondante en aminoacides, qui a été synthétisé selon le procédé de phase liquide ou

b) on traite par HF liquide anhydre un polypeptide de séquence correspondante en aminoacides lié à une résine porteuse, qui a été synthétisé selon le procédé de phase solide.

9. Préparations pharmaceutiques à administrer par voie parentérale, caractérisées par une teneur thérapeutiquement efficace en un polypeptide selon l'une des revendications 1—4 et par un véhicule inerte.

10. Préparations selon la revendication 9, caractérisées en ce que le véhicule est de l'albumine de sérum humain.

**Revendications pour l'Etat Contractant: AT**

1. Procédé de préparation de polypeptides de formule générale

(Leu$^{27}$)-GRF

dans laquelle GRF représente au moins les 28 premiers aminoacides de la séquence complète du facteur déclenchant la sécrétion de l'hormone de croissance humain naturelle et dans laquelle l'extrémité carboxy de la molécule est sous forme libre ou sous forme d'amide caractérisé en ce qu'

a) on élimine de la manière connue en soi les groupes protecteurs d'un polypeptide protégé de séquence correspondante en aminoacides, qui a été synthétisé selon le procédé de phase liquide ou

b) on traite par HF liquide anhydre un polypeptide de séquence correspondante en aminoacides lié à une résine porteuse, qui a été synthétisé selon le procédé de phase solide.

2. Procédé selon la revendication 1 de préparation de GRF, qui comprend les 41 à 44 premiers aminoacides du facteur déclenchant la sécrétion de l'homme de croissance humaine naturelle.

3. Procédé selon la revendication 1 de préparation de (Leu$^{27}$)-GRF (1—44)-OH.

4. Procédé selon la revendication 1 de préparation de (Leu$^{27}$)-GRF (1—44)-$NH_2$.

5. Préparations pharmaceutiques à administrer par voie parentérale, caractérisées par une teneur thérapeutiquement efficace en un polypeptide qui est préparé selon un procédé des revendications 1—4, et par un véhicule inerte.

6. Préparations selon la revendication 5, caractérisées en ce que le véhicule est de l'albumine de sérum humain.

7. Utilisation d'un polypeptide, qui a été préparé selon un procédé des revendications 1—4 pour l'application en tant que moyen accélérateur de croissance chez les animaux.

**Claims for the Contracting States: BE CH DE FR GB IT LI LU NL SE**

1. Polypeptides of the general formula

(Leu$^{27}$)-GRF

wherein GRF represents at least the first 28 amino acids of the full sequence of natural human growth hormone releasing factor and wherein the carboxyl terminus of the molecule is present in free form or as the amide.

2. A polypeptide according to claim 1, wherein GRF embraces the first 41 to 44 amino acids of natural human growth hormone releasing factor.

3. A polypeptide according to claim 1 having the formula ($Leu^{27}$)-GRF(1—44)-OH.

4. A polypeptide according to claim 1 having the formula ($Leu^{27}$)-GRF(1—44)-$NH_2$.

5. A polypeptide according to any one of claims 1—4 for use as a therapeutically active substance.

6. A polypeptide according to any one of claims 1—4 for use as a growth regulator in humans.

7. A polypeptide according to any one of claims 1—4 for use as a growth-promoting agent in animals.

8. A method for the preparation of polypeptides of the general formula

($Leu^{27}$)-GRF

wherein GRF represents at least the first 28 amino acids of the full sequence of natural human growth hormone releasing factor and wherein the carboxyl terminus of the molecule is present in free form or as the amide, characterized in that

(a) the protecting groups of a protected polypeptide of corresponding amino acid sequence, which has been synthesized according to the liquid phase method, are cleaved off in a manner known per se, or

(b) a carrier resin-bound protected polypeptide of corresponding amino acid sequence, which has been synthesized according to the solid phase method, is treated with anhydrous liquid HF.

9. A pharmaceutical preparation for parenteral administration, characterized by a therapeutically effective content of a polypeptide according to any one of claims 1—4 and an inert carrier material.

10. A preparation according to claim 9, characterized in that the carrier material is human serum albumin.

**Claims for the Contracting State: AT**

1. A method for the preparation of polypeptides of the general formula

($Leu^{27}$)-GRF

wherein GRF represents at least the first 28 amino acids of the full sequence of natural human growth hormone releasing factor and wherein the carboxyl terminus of the molecule is present in free form or as the amide, characterized in that

(a) the protecting groups of a protected polypeptide of corresponding amino acid sequence, which has been synthesized according to the liquid phase method, are cleaved off in a manner known per se, or

(b) a carrier resin-bound protected polypeptide of corresponding amino acid sequence, which has been synthesized according to the solid phase method, is treated with anhydrous liquid HF.

2. A method according to claim 1 for the preparation of GRF which embraces the first 41 to 44 amino acids of natural human growth hormone releasing factor.

3. A method according to claim 1 for the preparation of ($Leu^{27}$)-GRF(1—44)-OH.

4. A method according to claim 1 for the preparation of ($Leu^{27}$)-GRF(1—44)-$NH_2$.

5. A pharmaceutical preparation for parenteral administration, characterized by a therapeutically effective content of a polypeptide which has been prepared according to the process of claims 1—4 and an inert carrier material.

6. A preparation according to claim 5, characterized in that the carrier material is human serum albumin.

7. The use of a polypeptide which has been prepared according to any of the procsses of claims 1—4 for use as a growth-promoting agent in animals.